# DEMANDE DE BREVET EUROPEEN

(11) **EP 4 166 612 A1**
(43) Date de publication de la demande: **19.04.2023**
(21) Numéro de dépôt: 22202335.0
(22) Date de dépôt: 18.10.2022
(51) Int. Cl.: C08L 67/04, D01F 6/62, D01F 1/10, B33Y 70/00, C12P 7/625

(54) **COMPOSITIONS À BASE DE POLYHYDROXYALCANOATE(S) POUR LA RÉALISATION DE FILAMENTS D'IMPRESSION 3D, FORMULATIONS COMPRENANT CESDITES COMPOSITIONS, PROCÉDÉ DE PRÉPARATION DESDITES FORMULATIONS ET FILAMENT CONSOMMABLE POUR IMPRESSION 3D CORRESPONDANTS**

(30) Priorité: 18.10.2021 FR 2111063; 03.12.2021 FR 2112967
(71) Demandeur: Polymaris Biotechnology, 29200 Brest (FR)
(72) Inventeur: COURTOIS, Anthony, 29200 BREST (FR); THOLLAS, Bertrand, 29200 BREST (FR)
(74) Mandataire: Vidon Brevets & Stratégie

(57) **Abrégé**

L'invention concerne des formulations et compositions de filaments consommables pour impression 3D comprenant au moins polyhydroxyalcanoate. L'invention concerne également un procédé de préparation de telles formulations et compositions. L'invention concerne encore des filaments consommables pour impression 3D comprenant de telles formulations.

## Description

### Domaine technique

La présente invention appartient au domaine technique de l'impression 3D, et plus précisément des formulations et compositions utilisables pour des filaments d'impression 3D.

Plus particulièrement, l'invention concerne des formulations et compositions de filaments consommables pour impression 3D comprenant au moins un polyhydroxyalcanoate. L'invention concerne également un procédé de préparation de telles formulations et compositions. L'invention concerne encore des filaments consommables pour impression 3D réalisés selon ces formulations.

### Art antérieur

Les polyhydroxyalcanoates (PHA) sont des polyesters de type thermoplastiques naturels qui peuvent être utilisés en remplacement des plastiques conventionnels dans différents domaines d'application, tels que les emballages de consommation, les doublures de couches jetables et les sacs poubelles, les produits alimentaires et médicaux, etc. Les efforts initiaux se sont concentrés sur leur utilisation pour des applications de moulage ou de thermoformage, en particulier pour des articles d'emballage de consommation tels que des bouteilles, des récipients pour cosmétiques, des stylos ou encore des tees de golf.

Une des propriétés les plus utiles des PHA, qui les distingue facilement des polymères dérivés de la pétrochimie, est leur origine naturelle et leur biodégradabilité, par exemple en compost domestique, industriel, dans les eaux douces ou dans l'eau de mer, et plus généralement dans tout milieu dans lequel un écosystème vivant et actif est présent.

La vitesse de biodégradation de ces PHA va dépendre de différents facteurs. La présence de microorganismes, la température, le pH, le poids moléculaire et la cristallinité en sont les principaux. Les PHA sont synthétisés naturellement par certains microorganismes lorsque l'environnement naturel est défavorable. Ce sont des molécules de réserves permettant au microorganisme de survivre.

La production de ces PHA par voie biotechnologique utilise notamment des biofermenteurs qui permettent de contrôler parfaitement les conditions de production de ce biopolymère.

La structure chimique d'un PHA est un polyester caractérisé par la longueur de la chaîne latérale en nombre de carbone. Il existe donc différentes structures chimiques de PHA :
- les PHA scl (*short chain length*), qui comprennent de 3 à 5 carbones ;
- les PHA mcl (*medium chain length*), qui comprennent de 6 à 14 carbones ; et
- les PHA lcl (*long chain length*), qui comprennent un nombre supérieur à 14 carbones.

Il existe actuellement des formulations pour la fabrication de filaments 3D à base de compositions de biopolymères contenant des PHA. Ces compositions connues de biopolymères pour filaments 3D contiennent principalement des PHB (PHA scl ayant 4 carbones) utilisés en faibles quantités, en mélange avec d'autres polymères qui ne sont pas des biopolymères (acide polylactique (PLA) principalement) ou des additifs (charges et fibres principalement). Ces formulations de l'art antérieur comprennent par exemple des compositions constituées de 20% de PHA pour 80% de PLA.

La demande internationale WO 2016/058097 A1 propose notamment des formulations pour la fabrication de filaments 3D, dans un but d'amélioration de la biodégradabilité desdits filaments. Ces formulations sont à base de PHA, et comprennent également toujours d'autres composés, tels que des plastifiants, des agents durcissants ou encore des agents nucléants. Ces composés additionnels peuvent être présents dans une formulation jusqu'à une teneur de 50% en poids, par rapport au poids total de ladite formulation. Les formulations divulguées dans ce document comprennent toujours un seul type de PHA.

Également, l'article « Evaluation of 3D-Printing Scaffold Fabrication on Biosynthetic Medium-Chain-Length Polyhydroxyalkanoate Terpolyester as Biomaterial-Ink" de Panaksri Anuchzn et Al. divulgue la fabrication de films à base de PHA mcl afin de réaliser des filaments pour impression 3D, à destination du milieu de l'ingénierie tissulaire. Ce document fait état de l'utilisation d'un seul terpolymère PHA mcl pour une composition d'impression, bien que le monomère principal de ce PHA soit constitué de trois éléments.

Encore, l'article « Additive manufacturing of polyhydroxyalkanoates (PHAs) biopolymers : Materials, printing techniques, and applications " de Mehrpouya Mehrshad et Al. divulgue l'utilisation de PHA dans des compositions destinées à l'impression 3D. Ces composés PHA sont notamment utilisés pour leur biodégradabilité, leur haute compatibilité et leur disponibilité à partir de ressources renouvelables. Les compositions divulguées dans cet article comprennent toujours un seul type de PHA, et il est proposé de mélanger ce PHA avec d'autres polymères et/ou fibres naturelles, afin d'apporter des propriétés spécifiques aux filaments 3D finaux utilisés dans le processus d'impression. Contre toute attente, les inventeurs de la présente invention ont réussi à mettre au point des filaments 3D composés majoritairement de biopolymères PHA, voir exclusivement, ce qui présente notamment un intérêt puisque ces biopolymères sont beaucoup mieux compostables et biodégradables que les PLA par exemple. Les PHA sont en effet biodégaradables dans les sols, en eau douce, en eau de mer, en compost domestique et en compost industriel, tandis que le PLA est dégradable uniquement en compost industriel. Ces PHA peuvent être intégrés soit seuls en formulation dans la fabrication de ces filaments, soit avec d'autres polymères distincts des PHA, les PHA restant en quantités majoritaires, et le cas échéant avec des fibres et/ou charges, en proportions minoritaires également. Les filaments 3D selon l'invention PHA peuvent comprendre au moins deux types distincts de PHA.

### Exposé de l'invention

L'invention consiste donc, d'une part, en des formulations utilisant des compositions de PHA comme base, ou en d'autres termes comme matière première, pour la fabrication de filaments pour impression 3D. Ces formulations comprennent de telles compositions de PHA en tant qu'ingrédient majoritaire, c'est-à-dire que ces compositions sont présentes dans les formulations de l'invention selon une teneur supérieure à 50% en poids, par rapport au poids total des formulations, allant jusqu'à 100% en poids, par rapport au poids total des formulations, préférentiellement une teneur supérieure à 60% en poids, par rapport au poids total de ladite formulation, plus préférentiellement une teneur supérieure à 70% en poids, par rapport au poids total de ladite formulation. Ainsi, de telles formulations comprennent au moins un PHA en tant qu'ingrédient majoritaire. Au sens de l'invention, on comprendra donc qu'une formulation comprend une composition telle qu'introduite précédemment, avec ou sans additifs. Dans un cas où aucun additif n'est ajouté à la formulation, celle-ci correspondra en tout point à la composition qu'elle comprend.

Une technique d'impression 3D qui met en œuvre ces formulations est, par exemple, l'impression 3D par dépôt de matière fondue, aussi appelée impression 3D FFF (Fused Filament Fabrication), qui consiste à déposer un matériau fondu, type plastique, par couches successives via une extrudeuse qui se déplace et construit un objet physique en suivant un dessin défini par un fichier de conception assisté par ordinateur.

L'invention consiste également, d'autre part, en des compositions constituées de PHA, ayant pour but d'être intégrées de façon majoritaire dans la fabrication de formulations pour filaments pour impression 3D. De telles compositions peuvent comprendre soit un seul type de PHA, soit un mélange de plusieurs types de PHA selon des ratios prédéterminés, soit un seul type de PHA et au moins un autre polymère et/ou biopolymère et/ou autre composé, distincts des PHA, soit un mélange de plusieurs types de PHA et au moins un autre polymère et/ou biopolymère et/ou autre composé, distincts des PHA. Au sens de l'invention, on comprendra donc qu'une composition comprend dans toutes les cas possibles du PHA, soit sous la forme d'un seul type de PHA, soit sous la forme d'un mélange d'au moins deux types de PHA, et qu'elle peut additionnellement comprendre un ou plusieurs autres polymères, qu'ils soient biosourcés ou non, voir un ou plusieurs autres composés, mais quoi qu'il en soit toujours distincts des PHA.

Ainsi, un premier objet selon l'invention concerne une composition destinée à la conception de filaments consommables pour impression 3D, laquelle comprend majoritairement au moins un polyhydroxyalcanoate (PHA).

L'invention repose dès lors sur une approche tout à fait nouvelle et originale de fabriquer des filaments pour impression 3D. De telles compositions selon l'invention comprenant des PHA en tant qu'ingrédients majoritaires, biopolymères d'origine naturelle et biodégradables, sont avantageuses d'un point vue écologique et économique.

Selon un mode de réalisation, l'au moins un PHA est choisi parmi le groupe comprenant : PHA scl, PHA mcl, PHA lcl, et leur combinaison.

L'utilisation possible de ces différents types de PHA permet de moduler les paramètres physiques, chimiques et mécaniques, entre autres, des compositions au regard des divers systèmes de fabrication additive dans lesquels elles peuvent être utilisées, des divers objets imprimables, ainsi que de l'application associée à ces objets imprimés.

Selon un mode de réalisation, l'au moins un PHA est issu de bactéries choisies parmi le groupe comprenant les bactéries de souche CNCM I-5658, les bactéries de souche CNCM I-5079, et leur combinaison.

Les PHA intégrés dans les compositions selon l'invention sont donc obtenus de façon classique par biosynthèse avec un microorganisme, ce qui n'engendre aucune difficulté particulière dans leur préparation.

Un deuxième objet selon l'invention concerne une formulation matière première pour la conception de filaments consommables pour impression 3D, laquelle comprend une composition telle que définie précédemment selon une teneur supérieure à 50% en poids, par rapport au poids total de la formulation, jusqu'à 100% en poids, par rapport au poids total de la formulation, préférentiellement une teneur supérieure à 60% en poids, par rapport au poids total de la formulation, plus préférentiellement une teneur supérieure à 70% en poids, par rapport au poids total de la formulation. Les compositions précédemment introduites permettent d'être intégrées à la production des formulations pour impression 3D. De telles formulations présentent, entre autres, les mêmes avantages que ceux des compositions qu'elles comprennent.

Selon un mode de réalisation, une telle formulation comprend en outre au moins un polymère additionnel, distinct d'un PHA.

Un tel polymère additionnel permet également de moduler les propriétés physiques, chimiques et mécaniques, entre autres, des formulations selon l'invention. La présence de tels composés peut par exemple avoir un impact sur le comportement physique ou chimique du ou des PHA présents dans les compositions selon l'invention, elles-mêmes présentes dans les formulations selon l'invention, notamment lors du chauffage de ces PHA pendant le processus d'extrusion d'impression 3D : la température de cristallisation du ou des PHA peut être par exemple modifiée par la présence de tels polymères additionnels.

Selon un mode de réalisation, une telle formulation comprend en outre au moins un additif.

Selon un mode de réalisation, cet au moins un additif est choisi parmi les fibres, les charges et leur combinaison.

Il est donc avantageusement possible de rajouter différents types d'additifs pour moduler les formulations et compositions selon l'invention, afin que celles-ci soient davantage adaptées à une extrusion de filament 3D.

Selon un mode de réalisation, une telle formulation comprend une teneur de 0,01% à 49% en poids de fibres, par rapport au poids total de la formulation, préférentiellement une teneur de 0,1% à 5% en poids, par rapport au poids total de la formulation, plus préférentiellement une teneur de 0,3% à 3% en poids, par rapport au poids total de la formulation, et/ou une teneur de 0,01% à 49% en poids de charges, par rapport au poids total de la formulation, préférentiellement une teneur de 1% à 30% en poids, par rapport au poids total de la formulation, plus préférentiellement une teneur de 10% à 20% en poids, par rapport au poids total de la formulation.

Ces gammes de valeurs acceptables pour l'intégration d'additifs dans les formulations selon l'invention permettent d'adapter encore davantage les formulations et compositions de l'invention en fonction de l'environnement d'impression 3D et des propriétés recherchées pour les objets imprimés.

Un troisième objet selon l'invention concerne un procédé de préparation d'une formulation telle que définie précédemment, comprenant les étapes suivantes :
- culture de bactéries productrices de PHA ;
- concentration d'une biomasse cellulaire créée par la culture de bactéries ;
- séchage de la biomasse cellulaire ;
- extraction du PHA de la biomasse ; et
- purification du PHA.

Cette formulation comprend une teneur supérieure à 50% en poids d'une composition telle que définie précédemment, par rapport au poids total de la formulation, jusqu'à 100% en poids, par rapport au poids total de la formulation, préférentiellement une teneur supérieure à 60% en poids, par rapport au poids total de la formulation, plus préférentiellement une teneur supérieure à 70% en poids, par rapport au poids total de la formulation.

Ce procédé, qui permet de préparer une formulation selon l'invention, met en œuvre des étapes classiques d'obtention de PHA, par la voie microbiologique, qui ne nécessitent nullement un matériel difficile à se procurer, et ne présente aucune difficulté particulière.

Selon un mode de réalisation, l'au moins un PHA est choisi parmi le groupe comprenant : PHA scl, PHA mcl, PHA lcl, et leur combinaison.

Selon un mode de réalisation, la formulation préparée selon ce procédé comprend en outre au moins un polymère additionnel, distinct d'un PHA.

Selon un mode de réalisation, une telle formulation comprend en outre au moins un additif, celui-ci étant choisi parmi les fibres et les charges.

Un quatrième objet selon l'invention concerne un filament consommable pour impression 3D comprenant une composition telle que définie précédemment ou une formulation telle qu'également définie précédemment.

### Description détaillée de l'invention

Il peut ainsi être préparé des formulations pour la fabrication de filaments pour impression 3D comprenant majoritairement des biopolymères, et notamment des PHA. De telles formulations peuvent comprendre une composition comprenant par exemple du PHBHV seul, où PHBHV correspond à des PHA scl avec 4 ou 5 carbones, ou bien une composition comprenant par exemple un mélange PHBHV et PHO, où PHO correspond à des PHA mcl avec 8 carbones. Dans tous les cas, de telles formulations peuvent comprendre en outre un ou plusieurs polymères et/ou biopolymères additionnels distinct des PHA, ainsi qu'une ou plusieurs charges et/ou fibres.

### Formulations selon l'invention

Une formulation pour filaments 3D selon l'invention peut ainsi être constituée soit seulement d'une composition comprenant uniquement un ou plusieurs PHA, soit d'une composition comprenant un ou plusieurs PHA en quantités majoritaires et comprenant en outre un ou plusieurs polymères distincts des PHA, voire un ou plusieurs autres composés, toujours distincts des PHA, soit de l'un ou l'autre de ces deux types de compositions en présence également d'une ou plusieurs charges et/ou fibres, ces dernières étant présentes dans la formulation en quantités minoritaires.

Les formulations conformes à l'invention peuvent ainsi comprendre au moins une fibre.

Par "fibre", il faut comprendre un objet de longueur L et de diamètre D tel que L soit très supérieure à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150. Les fibres utilisables dans les formulations de l'invention peuvent être des fibres d'origine synthétique ou naturelle, minérale ou organique. Elles peuvent être courtes ou longues, unitaires ou organisées par exemple tressées, creuses ou pleines. Leur section peut être quelconque et notamment de section circulaire ou polygonale (carrée, hexagonale ou octogonale) selon l'application spécifique envisagée. Leur forme peut être linéaire, courbe, sinusoïdale ou frisée. En particulier, leurs extrémités sont épointées et/ou polies pour éviter de se blesser. Elles peuvent notamment être du marc de fruits, par exemple du marc de pomme, ou encore de la cellulose. Cette dernière présente l'avantage de résister à de fortes températures.

Lorsqu'elles sont présentes, les fibres peuvent être présentes dans les formulations selon l'invention en une teneur allant de 0,01% à 49% en poids, par rapport au poids total de la formulation, en particulier de 0,1% à 5% en poids, et plus particulièrement de 0,3% à 3% en poids.

Les formulations conformes à l'invention peuvent également comprendre au moins une charge. Les charges peuvent être choisies parmi celles bien connues de l'homme du métier et couramment utilisées dans les formulations pour la fabrication de filaments 3D. Ces charges peuvent être inertes ou conférer à la composition des activités biologiques. Elles peuvent notamment être de la poudre de coquille d'huître, du kaolin calciné, ou encore du carbonate de calcium.

Lorsqu'elles sont présentes, les charges peuvent être présentes dans les formulations selon l'invention en une teneur allant de 0,01% à 49% en poids, par rapport au poids total de la formulation, en particulier de 1% à 30% en poids, et plus particulièrement de 10% à 20% en poids.

L'homme du métier est en mesure de choisir, parmi l'ensemble de ces éventuels additifs, aussi bien la nature que la quantité de ceux qui seront ajoutés aux formulations, de telle sorte que celles-ci conservent l'ensemble de leurs propriétés. Bien entendu, l'homme du métier veillera à choisir les éventuels additifs complémentaires et/ou leur quantité de telle manière que les propriétés avantageuses des formulations selon l'invention ne soient pas, ou substantiellement pas, altérées par l'adjonction envisagée.

Notamment, les charges et fibres utilisées dans l'invention sont des composés qui ont pour but d'améliorer la processabilité des formulations qui rentrent dans la fabrication de filaments pour impression 3D. Elles sont intégrées dans ces formulations pour agir sur des caractéristiques prédéterminées des filaments que l'on cherche à obtenir, c'est-à-dire par exemple sur la dureté de ces filaments, leur température de fusion, leur température de cristallisation ou encore leur transition vitreuse. Ces charges et fibres sont utilisées dans les formulations de l'invention pour les effets mécaniques qu'elles induisent sur les filaments produits, mais également pour d'autres effets possibles, tels que des effets esthétiques. Dans ce sens, il est possible d'intégrer à ces formulations des charges qui sont des colorants : de tels composés colorants peuvent être utilisés dans l'invention aussi bien pour les propriétés mécaniques qu'ils induisent aux filaments que pour l'effet esthétique de coloration qu'ils apportent. Ils peuvent également être utilisés uniquement pour l'effet de coloration. Les charges et fibres utilisées dans l'invention peuvent également être des agents nucléants (charges), lesquels, lorsqu'ils sont intégrés aux formulations de l'invention, permettent aux filaments produits de durcir/de se figer plus rapidement, car ils agissent sur la température de cristallisation du ou des PHA présent dans ces formulations. Cet additif permet dès lors aux filaments produits de ne pas coller et de pouvoir être enroulés sur eux-mêmes pour former des bobines de filaments.

Les charges et fibres selon l'invention peuvent également conférer des propriétés biologiques aux filaments produits. Par exemple, l'intégration de cuivre dans les formulations de l'invention peut conférer des propriétés antibactériennes aux filaments.

Les formulations selon l'invention peuvent encore conférer des propriétés mécaniques différentes au sein d'une même bobine de filaments, en y intégrant différentes charges et/ou fibres conférant chacune une propriété mécanique distincte (par exemple tout à la fois propriété d'aimantation, propriété antibactérienne et action sur la température de cristallisation du ou des PHA de la composition de la formulation).

L'intégration dans les formulations selon l'invention de différentes charges et/ou fibres peut engendrer également un effet synergique sur les propriétés finales des filaments produits. En effet, le mélange de charges et/ou fibres distinctes au sein d'une même formulation peut améliorer chacun des effets apportés par ces charges et/ou fibres par rapport à ces effets présents isolément dans des formulations séparées. Un tel mélange de charges et/ou fibres peut apporter également à une formulation un effet nouveau aux filaments produits, qui résulte de l'action simultanée de ces charges et/ou fibres.

### Compositions selon l'invention

Une composition de PHA intégrable dans une formulation pour filaments 3D selon l'invention peut donc comprendre soit un seul type de PHA (par exemple du PHA scl, tel que du PHBHV ou du PHB), soit un mélange d'au moins deux types de PHA (par exemple un mélange PHA scl/PHA mcl, tel que PHBHV/PHO, ou encore deux types de PHA scl tel que PHBHV/PHB).

Le ratio PHA scl/PHA mcl, par exemple PHBHV/PHO, dans les compositions selon l'invention peut aller de 50/50 jusqu'à 99,9/0,1, préférentiellement de 70/30 à 99/1, plus préférentiellement de 90/10 à 95/5.

Le ratio PHBHV/PHB dans les compositions selon l'invention peut aller de 50/50 jusqu'à 99,9/0,1 préférentiellement de 60/40 à 99/1, plus préférentiellement de 80/20 à 95/5.

Naturellement, les bornes de ces intervalles ne sont pas des bornes impératives, et l'Homme du Métier pourra ajuster les valeurs préférées autour de 80/20, 90/10 ou 95/5.

Pour mémoire, comme indiqué plus avant dans la présente demande, il est également possible de prévoir un seul type de PHA, par exemple du PHBHV seul, donc à une teneur de 100%.

Ces compositions de PHA selon l'invention peuvent également comprendre en outre au moins un polymère additionnel ou autre composé additionnel, distinct des PHA et présent en proportions minoritaires dans les compositions. Ces polymères et/ou molécules additionnel(e)s peuvent être également des biopolymères, bien que distincts des PHA. Ils peuvent notamment être choisis parmi le poly(succinate de butyle) (PBS), le polycaprolactone (PCL), le poly(butylène succinate-co-butylène adipate) (PBSA), le polytéréphtalate de triméthylène (PTT), l'acide polylactique (PLA), l'amidon, le téréphtalate d'adipate de polybutylène (PBAT), le polyuréthane (PU), les polysaccharides (PS), ou encore les exopolysaccharides (EPS), cette liste n'étant pas exhaustive.

De telles formulations et compositions selon l'invention n'ont jamais été réalisées jusqu'à aujourd'hui, car le PHA principalement développé, le PHB, est un biopolymère cassant et incompatible seul pour cette application, d'où la préparation et l'utilisation dans l'art antérieur d'une formulation contenant une faible proportion en PHB.

Il en effet courant que des polymères à haute masse moléculaire subissent le phénomène de « cristallisation froide », relatif, lors du refroidissement de ces derniers, au fait qu'ils ne parviennent pas à cristalliser à cause de leurs chaînes macromoléculaires trop longues. Ils se figent donc dans un état vitreux. Lorsqu'ils sont réchauffés, les chaînes de ces polymères acquièrent suffisamment d'énergie pour qu'elles se mettent à bouger, leur permettant ainsi d'acquérir une position permettant la cristallisation. Il s'agit alors d'une cristallisation imparfaite, qui se traduit par des propriétés mécaniques amoindries et des risques de déformation de la pièce imprimée, en raison d'une post-cristallisation qui se produit au cours du temps.

Dès lors, selon l'invention, lorsqu'il est utilisé du PHB seul dans les compositions de PHA, de telles compositions peuvent comprendre également au moins un autre type de PHA qui n'est pas du PHB, et/ou des polymères additionnels, tels que des biopolymères distincts des PHA. Également, une solution pour contrer cet effet cassant des filaments 3D, qui ne permet pas de réaliser des bobines de filaments, dû à la présence de PHB en grandes quantités dans les compositions de l'invention, les formulations finales qui comprennent de telle compositions peuvent comprendre également des charges et/ou des fibres.

En effet, dans la fabrication des filaments 3D, les PHA des compositions selon l'invention, qui sont des composés amorphes, sont fondus à une certaine température pour former une résine, puis refroidis pour permettre leur cristallisation. Par exemple, la température de cristallisation du PHA scl PHBHV est aux environs de 60°C. S'il est utilisé seul dans les compositions, sa cristallisation ne sera terminée que lorsque le filament produit aura atteint cette température d'environ 60°C, ce qui engendre que le filament produit peut se rétracter longtemps après sa production. Dès lors, lorsqu'il est utilisé du PHBHV comme unique PHA dans les compositions selon l'invention, il est intéressant, voire nécessaire, d'ajouter un autre polymère distinc des PHA, et/ou au moins une charge ou fibre à la formulation finale, tel qu'un agent nucléant (charge), ce qui permet de rendre la température de cristallisation du PHBHV négative, et donc sa cristallisation immédiate. Le filament produit ne se rétractera pas une fois sa production finalisée en sortie d'imprimante.

Ce genre de paramètres (température de cristallisation, de fusion, transition vitreuse, etc.) est mesurable par exemple par la technique de calorimétrie différentielle à balayage (Differential Scanning Calorimetry, DSC), qui permet de mesurer les températures et les flux de chaleur associées aux transitions thermiques d'un matériau.

En outre, une composition constituée uniquement d'un mélange de PHA scl et de PHA mcl se présente sous la forme d'une résine, et plus précisément, sous la forme de granulés. Ces granulés peuvent notamment être utilisés pour la fabrication de filaments d'impression 3D. Une telle composition ou résine présente l'avantage de pouvoir être adjuvée/formulée avec des fibres, des charges, ou encore des pigments, et garde toutes ses propriétés pour l'obtention de filaments 3D imprimables. L'incorporation de certaines charges possédant des propriétés, par exemple biologiques ou chimiques, permettent d'obtenir des filaments pour impression 3D avec ces activités.

### Obtention des biopolymères polyhydroxyalcanoates

La présente invention ne couvre donc pas expressément les souches capables de produire les biopolymères, mais l'utilisation des polyhydroxyalcanoates secrétés par celles-ci pour la réalisation de formulations et compositions pour la fabrication de fils pour impression 3D.

L'obtention du PHA scl est obtenu de façon connue par biosynthèse avec un microorganisme de genre *halomonas* par un procédé de fermentation, et en particulier le microorganisme enregistré à la Collection Nationale de Culture des Microorganismes sous le traité de Budapest sous le numéro CNCM I-5079, dépôt effectué au nom de la société Polymaris Biotechnology, sise à Brest - France. L'obtention du PHA mcl est également obtenu de façon connue par biosynthèse avec un microorganisme de genre *pseudomonas* par un procédé de fermentation, et en particulier, le microorganisme enregistré à la Collection Nationale de Culture des Microorganismes sous le traité de Budapest sous le numéro CNCM I-5658 dépôt effectué au nom de la société Polymaris Biotechnology, sise à Brest - France.

La fermentation pour produire le PHA est réalisée dans des conditions d'agitation et d'aération du milieu à une température comprise entre 20°C et 37°C, préférentiellement à 30°C, le milieu ayant un pH compris entre 6,5 et 9 préférentiellement proche de 7,5, et étant ajusté si nécessaire durant la fermentation. La durée de la fermentation est comprise entre 24 et 120 heures, préférentiellement comprise entre 36 et 72 heures. Selon un mode de réalisation de l'invention, des sels minéraux sont apportés durant la fermentation de la bactérie. A titre illustratif et non limitatif, on peut citer parmi les sels minéraux ceux aptes à fournir au milieu de culture des ions Na⁺, K⁺, NH₄⁺, Ca²⁺, Mg²⁺, PO₄³⁻ , SO₄²⁻, Cl⁻, CO₃⁻ ou des éléments essentiels tels que Cu, Mn, Fe et Zn.

Les méthodes d'isolation et de purification du PHA sont obtenues par des moyens classiques et bien connus de l'homme de l'art, tels que la centrifugation, la filtration, l'ultrafiltration, l'extraction, l'évaporation et la dialyse.

### Structure du PHA scl

Le PHA scl qui peut être intégré aux compositions selon l'invention consiste en une répétition d'unités polymériques de formule A1 et d'unités polymériques de formule B1, telles que :

Formule A1 : -[-O-CH(R1)-(CH2)n-CO-]-

Formule B1 : -[-O-CH(R2)-(CH2)m-CO-]-

dans lesquelles : R1 et R2, différents l'un de l'autre, représentent un groupe choisi parmi alkyle (C0-C2) linéaire ou ramifié, et alcényle (C0-C2) linéaire ou ramifié, ledit groupe alkyle ou alcényle étant optionnellement substitué par 1 à 4 groupes, identiques ou différents, choisis parmi halogène (F, Cl, Br, I), -CN, -OH, - OR, -NH₂, -NHR, CO₂H, -CO₂R dans lesquels R représente un groupe alkyle (C1-C6) linéaire ou ramifié ; n et m, identiques ou différents, représentent un entier choisi parmi 1 ou 2.

Dans le cas du P3HBHV : R1= C1, R2 =C2, avec n=m=1.

### Structure du PHA mcl

Le PHA mcl qui peut être intégré aux compositions selon l'invention consiste en une répétition d'unités polymériques de formule A1', d'unités polymériques de formule B1' et d'unités polymériques de formules C1 telles que :

Formule A1' : -[-O-CH(R1)-(CH2)n-CO-]-

Formule B1' : -[-O-CH(R2)-(CH2)m-CO-]-

Formule C1 : -[-O-CH(R3)-(CH2)p-CO-]-

dans lesquelles : R1, R2 et R3 différents les uns des autres, représentent un groupe choisi parmi alkyle (C3-C11) linéaire ou ramifié, et alcényle (C3-C11) linéaire ou ramifié, ledit groupe alkyle ou alcényle étant optionnellement substitué par 1 à 4 groupes, identiques ou différents, choisis parmi halogène (F, Cl, Br, I), -CN, -OH, -OR, -NH₂, -NHR, CO₂H, -CO₂R dans lesquels R représente un groupe alkyle (C1-C6) linéaire ou ramifié ; n, m et p, identiques ou différents, représentent un entier choisi parmi 1 ou 2.

Dans le cas du PHO : R1 = C3, R2 = C5, avec n=m=1, C1 n'existant pas.

### Préparation et isolation des polyhydroxyalcanoates

### Méthode de culture de la bactérie enregistrée sous le numéro de dépôt CNCM I-5658

Pour la production du biopolymère PHA, l'ensemble des opérations sont réalisées selon les bonnes pratiques de microbiologie en conditions stériles. Les différents milieux sont également stérilisés soit par traitement thermique (121°C pendant 20 minutes), soit par filtration 0,2µm.

Un cryotube de 2 mL de la souche CNCM I-5658 est inoculé dans un erlenmeyer contenant 50 mL de milieu permettant la culture de microorganismes marin (milieu marine broth E2216, Difco, France). La culture est incubée à 30°C sous agitation pendant 12 à 18 heures.

La culture obtenue est ensuite inoculée dans un erlenmeyer contenant 1,45 L de milieu minéral adapté à sa croissance (milieu M1) à 30°C sous agitation pendant 8 heures.

Enfin, la culture est inoculée en fermenteur contenant 13,5 L de milieu minéral de culture adaptée à sa croissance et la biosynthèse du biopolymère (milieu M2) contenant du dextrose (10 g/L). La culture est maintenue à 30°C sous agitation pendant au minimum 30 heures.

Pendant les 28 premières heures de culture, il est de manière régulière une solution d'acide octanoïque tamponnée à pH 6 avec du NaOH 10N.

Le pH est régulé à 7,2 par du NaOH 10 N ou du H₂S0₄ 2N.

L'oxygénation et l'agitation de la culture sont également régulées pour permettre la croissance des cellules et l'accumulation du biopolymère.

A la fin de la fermentation, la biomasse cellulaire contenant les biopolymères est concentrée par centrifugation.

Cette biomasse est ensuite séchée afin d'éliminer toute l'eau et jusqu'à obtention d'une masse constante.

La composition du milieu M1 est détaillée dans le tableau 1 ci-après.

**[Tableau 1]**

| Éléments | g/kg dans H₂O | g/kg dans H₂O |
|---|---|---|
| NaNH₄HPO_{4.}4H₂O | 3,5 | |
| KH₂PO₄ | 3,7 | |
| K₂HPO₄ | 7,5 | |
| MgSO₄ | 0,25 | |
| NaCl | 20 | |
| Solution n° 1A | 40 | |
| Citrate de sodium | | 250 |
| Extrait de levure | | 25 |
| Solution n°2 | 1,3 | |
| FeSO₄.7H₂O | | 2,78 |
| CaCl₂.2H₂O | | 1,47 |
| MnCl₂.4H₂O | | 1,98 |
| CoCl₂.6H₂O | | 2,38 |
| ZnSO₄.7H₂O | | 0,29 |
| CuCl₂.2H₂O | | 0,17 |

La composition du milieu M2 est détaillée dans le tableau 2 ci-après.

**[Tableau 2]**

| Éléments | g/kg dans H₂O | g/kg dans H₂O |
|---|---|---|
| NaNH₄HPO_{4.}4H₂O | 3,5 | |
| KH₂PO₄ | 3,7 | |
| K₂HPO₄ | 7,5 | |
| MgSO₄ | 0,25 | |
| NaCl | 20 | |
| Solution n° 1A | 50 | |
| Citrate de sodium | | 200 |
| Extrait de levure | | 2 |
| Solution n°2 | 1,0 | |
| FeSO₄.7H₂O | | 2,78 |
| CaCl₂.2H₂O | | 1,47 |
| MnCl₂.4H₂O | | 1,98 |
| CoCl₂.6H₂O | | 2,38 |
| ZnSO₄.7H₂O | | 0,29 |
| CuCl₂.2H₂O | | 0,17 |

### Méthode de culture de la bactérie enregistrée sous le numéro de dépôt CNCM I-5079

Pour la production du biopolymère, l'ensemble des opérations sont réalisées selon les bonnes pratiques de microbiologie en conditions stériles. Les différents milieux sont également stérilisés soit par traitement thermique (121°C pendant 20 minutes), soit par filtration 0,2µm.

Le milieu M3 est composé de 0,5 g/L de NH₄Cl, 0,5 g/L de MgSO·7H₂O, 0,1 g/L de CaCl₂·2 H₂O, 0,04 g/L de KH₂PO₄, 0,12 g/L de K₂HPO₄, 20 g/L de NaCl et 6 g/L de HEPES. Une solution d'éléments traces a également été préparée. Elle est constituée de 7,3 g/L de Na₂-EDTA, 2,5 g/L de FeSO₄·7H₂O, 0,02 g/L de MnCl₂·4H₂O, 0,242 g/L de Na₂MoO₄·2H₂O, 0,038 g/L de CuCl₂·2H₂O et est ajoutée après autoclavage au milieu de culture.

Un cryotube de 2 mL de la souche CNCM I-5079 est inoculé dans un erlenmeyer contenant 50 mL de milieu permettant la culture de microorganismes marin (milieu marine broth E2216, Difco, France). La culture est incubée à 30°C sous agitation pendant 12 à 18 heures.

La culture obtenue est ensuite inoculée dans un erlenmeyer contenant 1,45 L de milieu minéral adapté à sa croissance (milieu M3) à 30°C sous agitation pendant 8 heures.

Enfin, la culture est inoculée en fermenteur contenant 13,5 L de milieu minéral de culture adaptée à sa croissance et la biosynthèse du biopolymère (milieu M2 + glycérol). La culture est maintenue à 30°C sous agitation pendant au minimum 30 heures.

Au cours de la culture, il est ajouté du glycérol et de l'acide valérique pour permettre l'accumulation du PHA scl désiré.

Le pH est régulé à 7,2 par du NaOH 10 N ou du H₂S0₄ 2N.

L'oxygénation et l'agitation de la culture sont également régulées pour permettre la croissance des cellules et l'accumulation du biopolymère.

A la fin de la fermentation, la biomasse cellulaire contenant les biopolymères est concentrée par centrifugation.

Cette biomasse est ensuite séchée afin d'éliminer toute l'eau et jusqu'à obtention d'une masse constante.

### Purification des biopolymères de polyhydroxyalcanoates scl et mcl

L'extraction du biopolymère PHA est réalisée par ajout de dichlorométhane à raison de 10 % de biomasse sèche par volume de solvant à température ambiante et sous agitation pendant 18 heures. La biomasse est éliminée par centrifugation (8000 rpm X 20 min) et le surnageant contenant le biopolymère est récupéré.

Ce dernier peut être filtré afin d'améliorer la qualité du biopolymère final.

Il est concentré par élimination du dichlorométhane à l'aide d'un évaporateur rotatif.

Le biopolymère concentré en solution dans le dichlorométhane est enfin précipité par ajout d'une solution d'éthanol à froid (10 : 1) puis séché dans une étuve ventilée à 40°C jusqu'à évaporation complète du solvant.

### Caractérisation physico-chimique du biopolymère polyhydroxyalcanoate obtenu

Le poids moléculaire du PHA obtenu est déterminé par chromatographie d'exclusion stérique avec pour détection un réfractomètre (RI). Il est déterminé par comparaison du temps de rétention du polymère avec le temps de rétention de standards Polystyrène via une droite d'étalonnage.

L'analyse permet de déterminer le poids moléculaire moyen (Mw en Dalton), le poids moléculaire moyen en nombre (Mn en Dalton) en équivalent PS (polystyrène), ainsi que l'indice de polydispersité. La composition du biopolymère est déterminée par chromatographie en phase gazeuse munie d'un détecteur à ionisation de flamme (FID) et confirmée par analyse RMN 1H, 13C, HMBC et HMQC.

On a ainsi obtenu un copolymère consistant en 96% en poids de poly(hydroxy-3-octanoate) et 4% en poids de poly(hydroxy-3-hexanoate) pour le PHA mcl, et un copolymère de 80% de poly-3-hydroxybutyrate et 20 % de poly-3-hydroxyvalerate pour le PHA scl.

### Exemples

### Essais d'extrusion de filaments avec des formulations comprenant une composition de PHA et une charge, qui est un agent nucléant

Il a d'une part été testé la formation d'une bobine de filaments par extrusion filament en utilisant des formulations comprenant majoritairement une composition constituée de PHA, et en particulier du PHA scl, en mélange avec un agent nucléant. Un tel agent nucléant, de par ses effets de nucléation, participe au contrôle, voir à l'amélioration, des propriétés structurelles et mécaniques des filaments de PHA.

Il a été préparé trois formulations différentes, selon le mode opératoire décrit dans la description détaillée ci-avant, destinées à être testées sur une ligne d'extrusion en laboratoire pour impression 3D. Il a notamment été cherché à former des bobines de filaments à intégrer dans des machines d'impression 3D dont les joncs extrudés présentaient un diamètre de 1,75 mm pour chacune de ces trois compositions.

La première formulation testée (F1) comprend 97% d'une composition de PHA scl, qui est du PHBHV, ainsi que 3% de fibre de cellulose de particules de 30 µm. Le PHBHV est composé de 80% en poids de poly-3-hydroxyburyrate et 20 % en poids de poly-3-hydroxyvalerate.

La deuxième formulation testée (F2) comprend 97% d'une composition de PHBHV, ainsi que 3% d'un agent nucléant qu'est le nitrure de bore.

La troisième formulation testée (F3) comprend 97% d'une composition de PHBHV, ainsi que 3% de nitrure de bore et 3% d'un colorant (ici, du MMbleu).

### Conditions de tests

Une extrudeuse de petit diamètre (15 mm - 20D) a été utilisée, composée de cinq zones de chauffe. La cavité de sortie de la filière était de 3 mm, et du tirage à cylindre a été effectué dans le but d'arriver à un jonc de diamètre 1,75 mm.

L'extrudeuse était équipée d'une pompe à engrenage et tête à engrenage pour un débit régulier. Elle était montée sur un dispositif de levage pour régler le refroidissement de l'extrudât et d'un bac de refroidissement à eau par débordement thermo régulé, chaud ou froid, en boucle fermée.

La ligne était équipée d'un bobinoir à trois positions à couple constant, afin de fournir des tensions d'enroulement faibles.

Une position pour avoir peu de tension a été utilisée pour l'essai, selon les paramètres suivants : trancannage 40 tr/min, poste le plus éloigné du bac 50 tr/min.

L'étuvage a été fait dans une turbo étuve à 50 °C la nuit (minimum 8h ; maximum 12h).

Pendant l'extrusion filament, le bac de refroidissement matière était à la température de 8°C, et a été augmenté jusqu'à 50°C.

### Résultats

Les résultats des tests d'extrusion sur les trois formulations sont regroupés dans le tableau 3 ci-après.

**[Tableau 3]**

| Formula tions | Température des zones de chauffe de l'extrudeuse (°C) | Pression (bars) et vitesse de rotation de la vis (rpm) | Vitesse de tirage (m.min⁻¹) | Température du bac de refroidissement (°C) | Résultats |
|---|---|---|---|---|---|
| F1 : essai 1 | De 150 à 175 | / | / | 8 | -Filament de 2mm -Matière collante |
| F1 : essai 2 | De 150 à 175 | 20 43 | / | 50 | -Filament de 1,7mm -Matière moins collante que essai 1 |
| F1 : essai 3 | De 150 à 175 | 20 38 | 4,3 | 50 | -Filament de 1,75mm -Obtention d'une bobine de filament -Mais matière qui reste collante |
| F2 | De 150 à 175 | 19 38 | 4,3 | 50 | -Filament de 1,75mm -Obtention d'une bobine de filament -Matière moins collante que F1 essai 3 |
| F3 | De 150 à 175 | 11 38 | 4.3 | 50 | -Filament de 1,75mm -Obtention d'une bobine de filament |

Il ressort des résultats de ce tableau qu'il a été nécessaire de conduire plusieurs tests dans des conditions différentes (vitesse de rotation de la vis, pression, température du bac de refroidissement, etc.) pour réussir à obtenir une bobine de filament à partir de la formulation F1. Cependant, la matière du filament de cette bobine reste collante, et il est nécessaire de faire couler de l'eau sur les rouleaux de l'extrudeuse afin de limiter l'adhésion du filament sur ces rouleaux. Cette bobine ainsi obtenue est donc difficile à dérouler. Il a dès lors été déterminé que la formulation F1 n'est pas parfaitement adaptée pour faire de l'extrusion filament.

Il a pu être réutilisé les conditions expérimentales de l'essai numéro 3 de F1 pour tester la formulation F2. Il a pu également être obtenu une bobine de filament dont la matière est moins collante que celle du filament de l'essai 3 de F1. Il n'est ainsi plus nécessaire de faire couler de l'eau sur les rouleaux de l'extrudeuse. Il a donc été déterminé que cette formulation F2 se comporte bien en extrusion filament. Les mêmes conditions expérimentales ont encore été appliquées pour le test d'extrusion de la formulation F3. Il a également pu être obtenu une bobine. Le filament de la bobine est légèrement blanchi par endroits (adhésion aux rouleaux, dispersion inhomogène du colorant), et la circulation d'eau sur les rouleaux a été remise en place par rapport au test de la formulation F2. Malgré tout, il a été déterminé que la formulation F3 est adaptée à l'extrusion filament.

### Essais d'extrusion de filaments avec des formulations comprenant uniquement des compositions de

### PHA, ainsi qu'avec des formulations comprenant des compositions de PHA en présence de charges

Il a d'autre part été testé la formation de bobines de filaments par extrusion filament en utilisant des formulations comprenant toujours majoritairement des compositions de PHA, et notamment des compositions constituées d'un mélange de PHA scl et de PHA mcl, ainsi qu'en utilisant de telles compositions en mélange avec des charges.

Dans un premier temps, il a été préparé différentes compositions, en mode batch, afin de déterminer les paramètres optimaux pour l'obtention de filaments aptes à conduire de l'impression 3D.

Les conditions de tels essais d'extrusion filament sont détaillées dans le tableau 4 ci-après.

**[Tableau 4]**

| Compositions | Température de l'extrudeuse (°C) | Vitesse de rotation de la vis (rpm) | PHBHV (% massique) | PHO (% massique) |
|---|---|---|---|---|
| Essai 1 | 168 | 50 | 95 | 5 |
| Essai 2 | 168 | 50 | 90 | 10 |
| Essai 3 | 168 | 50 | 85 | 15 |
| Essai 1' | 178 | 50 | 90 | 10 |
| Essai 2' | 178 | 50 | 85 | 15 |
| Essai 3' | 178 | 50 | 70 | 30 |

Sur la base des résultats de ces compositions testées en extrusion filament, lesquels résultats ont également pris en compte la cinétique de cristallisation du produit extrudé, il a été préparé quatre formulations différentes, toujours selon le mode opératoire décrit dans la description détaillée ci-avant, destinées à être testées sur une ligne d'extrusion en laboratoire pour impression 3D.

La première formulation testée (F1') comprend 100% d'une composition constituée de 95% de PHBHV et 5% de PHA mcl, qui est du PHO. Le PHO est composé de 96 % en poids de poly-3-hydroxyoctanoate et 4 % en poids de poly-3-hydroxyhexanoate.

La deuxième formulation testée (F2') comprend 80% d'une composition constituée de 76% de PHBHV et 4% de PHO, ainsi que 20% de poudre de coquille d'huître (ostrecal 15).

La troisième formulation testée (F3') comprend 80% d'une composition constituée de 76% de PHBHV et 4% de PHO, ainsi que 20% de kaolin calciné (opacilite).

La quatrième formulation testée (F4) comprend 80% d'une composition constituée de 76% de PHBHV et 4% de PHO, ainsi que 20% de carbonate de calcium.

Les résultats des tests d'extrusion sur ces quatre formulations, sur une ligne extrusion filage monovis 3DEvo Advanced 1.0, sont regroupés dans le tableau 5 ci-après.

**[Tableau 5]**

| Formulations | Température des zones de chauffe de l'extrudeuse (°C) | Vitesse de rotation de la vis (rpm) | Résultats |
|---|---|---|---|
| F1' | De 155 à 175 | 200 | Filaments aptes à l'impression 3D obtenus |
| F2 ' | De 155 à 175 | 250 | Filaments aptes à l'impression 3D obtenus |
| F3' | De 155 à 175 | 250 | Filaments aptes à l'impression 3D obtenus |
| F4 | De 155 à 175 | 250 | Filaments aptes à l'impression 3D obtenus |

Il en résulte de ces essais d'extrusion que les quatre formulations F1' à F4 se comportent bien en extrusion filament. Elles ont pu être ensuite intégrées à des essais d'impression 3D, dans une imprimante µ-Delta, EmotionTech, titan Extruder 1,75 mm, Hotends E3D V6, Buse 0,5 et 0,8 mm, et ont permis la fabrication d'objets en 3D, tels que des pots, des contenants, ou encore des objets plus complexes, à l'image de figurines de bateaux.

## Revendications

1. Composition destinée à la conception de filaments consommables pour impression 3D, **caractérisée en ce qu'**elle est constituée uniquement d'un ou plusieurs polyhydroxyalcanoates (PHA).

2. Composition selon la revendication 1, **caractérisée en ce qu'**elle est constituée d'un mélange d'au moins deux types de PHA.

3. Composition selon la revendication 1 ou 2, **caractérisée** en ce ledit au moins un PHA est choisi parmi le groupe comprenant : PHA scl, PHA mcl, et leur combinaison.

4. Composition selon la revendication 3, **caractérisée en ce que** lesdits PHA sont présents dans la composition selon un ratio PHA scl/PHA mcl allant de 50/50 jusqu'à 99,9/0,1, préférentiellement de 70/30 à 99/1, plus préférentiellement de 90/10 à 95/5.

5. Composition selon l'une quelconque des revendications 1 à 4, **caractérisée en ce que** ledit au moins un PHA est issu de bactéries choisies parmi le groupe comprenant les bactéries de souche CNCM I-5658, les bactéries de souche CNCM I-5079, et leur combinaison.

6. Formulation matière première pour la conception de filaments consommables pour impression 3D, **caractérisée en ce qu'**elle comprend une composition selon les revendications 1 à 5 selon une teneur supérieure à 50% en poids, par rapport au poids total de ladite formulation, jusqu'à 100% en poids, par rapport au poids total de ladite formulation, préférentiellement une teneur supérieure à 60% en poids, par rapport au poids total de ladite formulation, plus préférentiellement une teneur supérieure à 70% en poids, par rapport au poids total de ladite formulation.

7. Formulation selon la revendication 6, **caractérisée en ce qu'**elle comprend en outre au moins un polymère additionnel, distinct d'un PHA.

8. Formulation selon la revendication 6 ou 7, **caractérisée en ce qu'**elle comprend en outre au moins un additif.

9. Formulation selon la revendication 8, **caractérisée en ce que** ledit au moins un additif est choisi parmi les fibres, les charges et leur combinaison.

10. Formulation selon la revendication 9, **caractérisée en ce qu'**elle comprend une teneur de 0,01% à 49% en poids desdites fibres, par rapport au poids total de la formulation, préférentiellement une teneur de 0,1% à 5% en poids, par rapport au poids total de ladite formulation, plus préférentiellement une teneur de 0,3% à 3% en poids, par rapport au poids total de ladite formulation, et/ou une teneur de 0,01% à 49% en poids desdites charges, par rapport au poids total de la formulation, préférentiellement une teneur de 1% à 30% en poids, par rapport au poids total de ladite formulation, plus préférentiellement une teneur de 10% à 20% en poids, par rapport au poids total de ladite formulation.

11. Procédé de préparation d'une formulation selon les revendications 6 à 10 comprenant les étapes suivantes :
- culture de bactéries productrices de PHA ;
- concentration d'une biomasse cellulaire créée par ladite culture de bactéries ;
- séchage de ladite biomasse cellulaire ;
- extraction dudit PHA de ladite biomasse ;
- purification dudit PHA ; **caractérisé en ce que** ladite formulation comprend une teneur supérieure à 50% en poids d'une composition selon les revendications 1 à 5, par rapport au poids total de la formulation, jusqu'à 100% en poids, par rapport au poids total de la formulation, préférentiellement une teneur supérieure à 60% en poids, par rapport au poids total de ladite formulation, plus préférentiellement une teneur supérieure à 70% en poids, par rapport au poids total de ladite formulation.

12. Procédé selon la revendication 11, **caractérisé en ce que** ledit au moins un PHA est choisi parmi le groupe comprenant : PHA scl, PHA mcl, PHA lcl, et leur combinaison.

13. Procédé selon la revendication 11 ou 12, **caractérisé en ce que** ladite formulation comprend en outre au moins un polymère additionnel, distinct d'un PHA.

14. Procédé selon l'une quelconque des revendications 11 à 13, **caractérisé en ce que** ladite formulation comprend en outre au moins un additif, ledit additif étant choisi parmi les fibres et les charges.

15. Filament consommable pour impression 3D comprenant une composition selon les revendications 1 à 5 ou une formulation selon les revendications 6 à 10.
